# EUROPÄISCHE PATENTSCHRIFT

(11) **EP 0 776 223 B1**
(45) Veröffentlichungstag und Bekanntmachung des Hinweises auf die Patenterteilung: **09.06.1999**
(21) Anmeldenummer: 94926169.7
(22) Anmeldetag: 10.08.1994
(51) Int. Cl.: A61M 1/34

(54) **ANORDNUNG ZUR ELIMINATION VON SUBSTANZEN AUS FLÜSSIGKEITEN, INSBESONDERE BLUT**
ARRANGEMENT FOR REMOVING SUBSTANCES FROM LIQUIDS, IN PARTICULAR BLOOD
SYSTEME PERMETTANT D'ELIMINER DES SUBSTANCES CONTENUES DANS DES LIQUIDES, NOTAMMENT DU SANG

(30) Priorität: 10.08.1993 AT 159993
(43) Veröffentlichungstag der Anmeldung: 04.06.1997
(73) Patentinhaber: Falkenhagen, Dieter, A-2104 Spillern (AT)
(72) Erfinder: FALKENHAGEN, Dieter, A-2104 Spillern (AT); SCHIMA, Heinrich, A-1020 Wien (AT); LOTH, Fritz, D-14513 Teltow (DE)
(74) Vertreter: Luderschmidt, Schüler & Partner GbR
(86) Internationale Anmeldenummer: EP9402644
(87) Internationale Veröffentlichungsnummer: WO9504559

(56) Entgegenhaltungen:
- EP-A- 0 143 178
- DE-A- 4 113 602
- FR-A- 2 472 936
- US-A- 3 619 423
- US-A- 3 963 613
- US-A- 3 979 284
- US-A- 4 024 059
- US-A- 5 078 885

## Beschreibung

Die Erfindung betrifft eine Anordnung zur Elimination von Substanzen aus Flüssigkeiten, insbesondere Blut, die mindestens einen an einen ersten Kreislauf, der die zu behandelnde Flüssigkeit aufweist, anschließbaren Membranfilter umfaßt, in dessen Sekundär- bzw. Filtratseite eine Adsorbentien enthaltende Suspension vorliegt, um die durch die Membran gelangenden Substanzen zu binden.

Die Elimination von pathophysiologisch relevanten Substanzen kann mit extrakorporalen Systemen durgeführt werden, wobei Membranfilter und Adsorptionsverfahren zum Einsatz kommen. Zu diesen Verfahren zählen insbesondere die Hämodialyse, die High-Flux-Hämodialyse, die Hämofiltration, die Membranplasmaseparation, Hämoperfusion und die Plasmaperfusion. Für spezielle Anwendungen, wie z.B. die Therapie des Leberversagens, sind auch hybride Systeme unter Einschluß von speziellen Zelltypen, wie z.B. Hepatozyten, in der klinischen Erprobung. Beispiele für die Anwendung derartiger Therapieverfahren sind die chronische Niereninsuffizienz, akute exogene Intoxikationen, akute und chronische Leberinsuffizienz, der Endotoxinschock, metabolische Störungen, wie Hyperlipidämien und Autoimmunerkrankungen. Bei den derzeit bekannten pathophysiologisch relevanten Substanzen handelt es sich um ein umfassendes Spektrum verschiedener Substanzen mit unterschiedlichen chemischen (proteinlipid - wassergebunden, hydrophil, hydrophob) bzw. physikalischen Eigenschaften (Molekülgröße, Molekulargewicht), weshalb verschiedene Verfahren zur Anwendung kommen müssen.

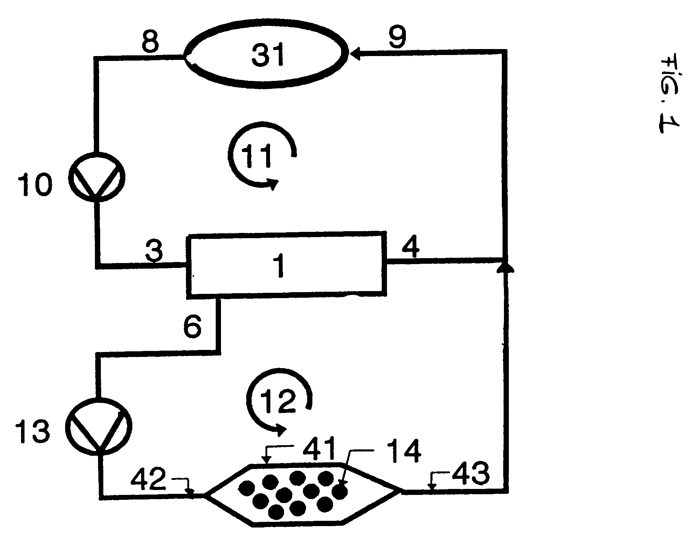

Um Substanzen, wie z.B. LDL-Cholesterol, Endotoxine, Antikörper, Antigene, protein/lipidgebundene Stoffe selektiv zu eliminieren, werden vorzugsweise adsorptionsgeschützte Verfahren angewandt. Nachteile derartiger Systeme sind:
- Schwierigkeit des Einsatzes in Form unmittelbar blutkontaktierender Systeme aufgrund der Blutverträglichkeits-Problematik, insbesondere bei feinteiligen Adsorbentien;
- Bei Plasmaperfusion, wie sie in Abb. 1 dargestellt ist, wird ein Filtrat aus dem Filter 1 über den Auslaß 6 in den Sekundärkreis 12 durch eine Pumpe 13 abgepumpt. Das filtrierte Plasma fließt sodann durch eine Kapsel 41, die die Wirkstoffe bindenden Partikel enthält, sodann fließt das gereinigte Plasma in den Primärkreis 11 und den Patienten 31 zurück. Dieses Verfahren bedingt einen hoben ökonomischen bzw. technologischen Aufwand, da aufgrund der begrenzten Bindungskapazität der Partikel die Kapsel 41 unter klinisch erforderlichen Bedingungen während des Einsatzes getauscht oder regeneriert werden muß.
- Während aufgrund der größeren aktiven Gesamtoberfläche die Verwendung möglichst kleiner Partikel anzustreben ist, führt die Verwendung derartiger kleiner Partikel in Kapseln 41 zu hohen Packungsdichten und damit zu hohen Flußwiderständen, die einen sehr hohen Druckabfall zwischen Einlaß 42 und Auslaß 43 bedingen.
- Darüber hinaus kommt es aufgrund der Plasmaentnahme im Filter 1 zu einer Eindickung des Blutes in der Nähe des Membranfilter-Auslasses 4 mit den damit verbundenen Risiken der Thrombenbildung.
- Da eine verläßliche mechanische Bindung der Partikel in der Kapsel 41 üblicherweise nicht garantiert werden kann, ist aus Sicherheitsgründen ein weiteres Filter vor dem Einlaß 9 in den Patienten vorzusehen.
- Bei Verwendung von Adsorbermaterialien enthaltenden Suspensionen wird bei den bekannten Verfahren (z.B. Biologic DT, PCT WO93/15825 Ash und US-Patent 5.078.885, Matsumura) vorrangig auf diffusiv ausgelegte Prinzipien Bezug genommen. Dabei werden entweder selektive Verfahren, wie die Anwendung asymmetrisch aufgebauter Membranen in Verbindung mit entsprechend geformten und daher sehr aufwendigen Adsorber-Partikeln vorgeschlagen oder die Verwendung aufwendiger Pumpsysteme zur pulsatilen Beförderung und Umwälzung der Suspension erörtert. Beide Verfahren sind mit hohen Kosten verbunden.

Eine Anordnung zur Elimination von Substanzen der im Oberbegriff des Patentanspruchs 1 genannten Art ist aus US-A-3,963,613 bekannt. Die bekannte Anordnung erlaubt es, mittels verschiedener Enzyme, die in Suspension vorliegen können oder verkapselt sind, unter Verwendung von Dialysat selektiv Substanzen zu entfernen. In einer bevorzugten Ausführungsform weist die bekannte Anordnung 2 parallel geschaltete Dialysatoren auf, wobei eine Kammer des zweiten Dialysators nicht in einen Flüssigkeitskreislauf eingebunden ist. Die bekannte Anordnung stellt ausschließlich auf die diffusive Elimination niedrigmolekularer Substanzen ab.

EP-A-0 143 178 beschreibt eine Anordnung zur Blutreinigung mit einem Filter, dessen Primärkompartiment in den Blutkreislauf geschaltet ist und dessen Sekundärkompartiment in einen Sekundärkreislauf geschaltet ist, in den eine Adsorberkapsel eingebunden ist. Ein Absorbentien enthaltende Suspension liegt im Sekundärkreislauf nicht vor. Die Erzeugung regional auftretender unterschiedlicher positiver und negativer Transmembrandrücke erfolgt ausschließlich durch Erhöhung der Förderrate des Blutes im Primärkreislauf.

Der Erfindung liegt die Aufgabe zugrunde, eine Anordnung der eingangs erwähnten Art zur Verfügung zu stellen, mit der die aus der Flüssigkeit, insbesondere Blut, zu entfernenden Substanzen mit hoher Selektivität und hoher Adsorptionsleistung entfernt werden.

Die Lösung der Aufgabe erfolgt erfindungsgemäß mit den Merkmalen des Patentanspruchs 1.

Bei der erfindungsgemäßen Anordnung wird das die zu eliminierenden Substanzen enthaltende Medium, insbesondere Blut, durch einen Membranfilter, insbesondere Plasmafilter oder Hämofilter, geleitet, der einerseits in eine Blutkammer und andererseits in eine Filtratkammer unterteilt ist. Dabei ist die Blutkammer die Primärseite, der üblicherweise Blut mit einer ersten Pumpe (peristaltischen Pumpe) zugeführt wird.

Die Filtratkammer, die die Sekundärseite bzw. die Filtratseite darstellt, ist mit einer Reinigungssuspension beaufschlagt, die Partikel aufweist, die die zu entfernenden Substanzen binden können. Diese Flüssigkeit weist üblicherweise weiterhin Elektrolyte in isotonischer Zusammensetzung auf.

Demzufolge wird also die Reinigungssuspension mit der Fördergeschwindigkeit einer zweiten Pumpe, die in den Sekundärkreislauf eingeschaltet ist, durch die Filtratkammer des Membranfilters hindurchgeführt.

Erfindungswesentlich ist weiterhin ein Mittel zur Erzeugung regional auftretender unterschiedlicher positiver und negativer Transmembrandrücke an der Membran. Dies läßt sich am besten an sog. High-Flux-Membranen durchführen, die in einer Vielzahl von Ausführungsformen am Markt sind. Diese zeichnen sich durch eine hohe Wasserdurchlässigkeit in Abhängigkeit von der Oberfläche der Membran, dem Druck und der Behandlungszeit aus. Derartige High-Flux-Filter haben für Wasser bei der Raumtemperatur eine Durchlässigkeit in einer Größenordnung von 15-20 ml/h x m² x mmHg und mehr. (1mmHg = 133.3 Pascal)

Aufgrund des Strömungsverhaltens der Reinigungssuspension an der Außenseite der üblicherweise als Hohlfaser ausgebildeten Membranfilter kommt es zu einem Druckabfall von der Eingangsseite zur Ausgangsseite der Filtratkammer, was zur Folge hat, daß bei ausreichend hohem Eingangsdruck, der durch die Fördergeschwindigkeit und den Strömungswiderstand des Mediums in der Filtratkammer im wesentlichen bestimmt wird, Flüssigkeit durch die Poren der Membran hindurch in die Blutbahn gedrückt werden.

Üblicherweise ist das Sekundärvolumen konstant, was zur Folge hat, daß auf der gegenüberliegenden Seite die gleichen Verhältnisse in umgekehrter Folge vorliegen, d. h. es kommt zu einer Rückfiltration (back filtration) des vorher durch die Membran geförderten Flüssigkeitsvolumens.

Vorteilhafterweise wird der Membranfilter im Gegenstrom betrieben, d.h. die beiden Flüssigkeiten werden durch die jeweilige Kammer in entgegengesetzter Richtung hindurchgefördert. Insofern treten jeweils am Eingang der Kammer positive Drücke auf, die am Ausgang der Kammer mit negativen Drücken korreliert sind. Bei der Reinigung von Blut führt dies dazu, daß Blutplasma zusammen mit den zu eliminierenden Substanzen auf der Bluteingangsseite durch die Membran hindurch in die Filtratseite gelangt, dort von den zu entfernenden Substanzen befreit wird und auf der gegenüberliegenden Seite wiederum wegen der Bilanzierung in den Blutkreislauf zurückkehrt.

Insofern ist es ebenfalls erfindungswesentlich, daß das Volumen des Sekundärkreislaufs bilanziert gehalten wird, was üblicherweise durch eine Konstanthaltung des Volumens des Sekundärkreislaufs erfolgt.

Vorteilhafterweise ist die Pumpe im Sekundärkreislauf mit dem Mittel zur Erzeugung unterschiedlicher Transmembrandrücke kombiniert, was dadurch erfolgt, daß die Pumpe mit relativ hohen Förderraten von 0,5-6 l/min, vorzugsweise 1-3/l mit den entsprechenden Eingangsdrücken betrieben wird.

Weiterhin ist vorteilhafterweise die zur Förderung der Suspension im Sekundärkreislauf vorgesehene Pumpe eine Zentrifugalpumpe, die sich durch eine niedrige mechanische Scherbelastung gegenüber den suspendierten Adsorptionspartikeln auszeichnet. Es kommt daher nur zu einer minimalen mechanischen Schädigung dieser Partikel beim Umpumpen.

Gemäß einer weiteren vorteilhaften Ausführungsform ist neben einem ersten Membranfilter ein zweiter Membranfilter in Reihe geschaltet, so daß die beiden Blutkammern bzw. Filtratkammern nacheinander von Blut bzw. Reinigungssuspension durchflossen werden. Diese Anordnung führt zu einer Erhöhung der Eliminationsleistung, insofern, als in die Verbindungsleitung zwischen den Filtern auf der Filtratseite eine einstellbare Klemme als weiteres Mittel zur Erzeugung unterschiedlicher Transmembrandrücke eingeschaltet sein kann.

Wie bereits vorstehend erwähnt, ist im Sekundärkreislauf eine Bilanziervorrichtung vorgesehen, mit der die zuzuführerden und abzuführenden Flüssigkeitsmengen gegeneinander bilanziert werden können. Eine solche Bilanziervorrichtung kann beispielsweise zwei gleichartig betriebene Fördereinrichtungen (Pumpen) aufweisen, von denen die eine im Zuführungsast und die andere im Abführungsast des Kreislaufs vorgesehen ist. Diese Pumpanordnung (beispielsweise eine Doppelschlauchpumpe) kann gleiche Mengen an Reinigungssuspension zu- und abführen. Andererseits kann jedoch aber auch ein Bilanzkammersystem zur Anwendung kommen, wie sie beispielsweise in der DE-28 38 414 beschrieben ist und in zahlreichen bilanzierten Hämodialysevorrichtungen zum Einsatz kommt.

Es ist bevorzugt, wenn die Reinigungssuspension nur so lange durch den Sekundärkreislauf hindurchgeführt wird, bis ihre Eliminationsleistung nahezu verbraucht ist. Dabei soll sich verbrauchte Reinigungssuspension möglichst nicht mit frischer Reinigungssuspension vermischen, was vorteilhafterweise durch ein Sperrventil erfolgt, das eine Durchmischung verhindert. Zusätzlich kann eine Pumpe zur Beschleunigung des Austauschs vorgesehen sein.

Sämtliche mit der biologischen Flüssigkeit, vorzugsweise Blut, in Berührung kommenden Teile sind nur für den einmaligen Verbrauch bestimmt (sog. Disposables), wobei beispielsweise die im Sekundärkreislauf eingesetzte Pumpe(n) aus einem wiedereinsetzbaren Antriebsteil und einem zur einmalige Verwendung bestimmten Pumperkopf bestehen.

Die erfindungsgemäße Anordnung verfügt weiterhin vorteilhafterweise über Detektoren zur Erkennung von Defekten des Filters, die in die Flüssigkeit des Primärkreislaufs gelangten. Zu solchen Detektoren gehören Ultraschalldetektoren, photooptische Sensoren, die eine photooptisch detektierbare Substanz ermitteln können, Magnetsensoren, die magnetisch aktive Substanzen ermitteln können, und dergleichen.

Gemäß einer weiteren Ausführungsform ist ein Maschenfilter nach dem Auslaß der Primärseite des Membranfilter geschaltet, um infolge eines Defekts durchgelassene Adsorptionspartikel sicher vor dem Eindringen in die Blutbahr eines Patienten zurückzuhalten.

Des weiteren kann im Primärkreislauf nach dem Auslaß der Primärseite des Membranfilters ein magnetisches Feld aufgebracht werden, was magnetisch aktive Substanzen, die in der Reinigungssuspension als Partikel vorgesehen sind, ablenkt, sofern diese Partikel bei einem Defekt des Membranfilters auf die Primärseite gelangt sind.

Es ist weiterhin vorteilhaft, wenn jeweils auf der Einlaß- und Auslaßseite der Kammern des Membranfilters Drucksensoren angebracht sind, so daß sich ohne weiteres der Transmembrandruck (TMP) über die gesamte Membran hinweg ermitteln läßt und damit auch die zweite Pumpebene, das Mittel zur Erzeugung unterschiedlicher Membrandrücke in vorbestimmter Weise steuern läßt.

Die in der Reinigungssuspension vorgesehenen Partikel sind entsprechend den zu eliminierenden Substanzen angepaßt. So läßt sich beispielsweise die erfindungsgemäße Anordnung besonders vorteilhaft zur Entfernung von Endotoxinen oder anderen Mediatoren einschließlich Zytokine, wie dem Tumor-Negrose-Faktor oder Interleukin 1, einsetzen. Die Partikel verfügen dabei über eine hohe Adsorptionskapazität, die durch eine hohe gutsmengenbezogene Oberfläche gekennzeichnet ist. Besonders vorteilhaft werden sog. Mikrokugeln (Microspheres) eingesetzt, die üblicherweise einen Durchmesser von 5-50 µm aufweisen.

Will man nun Endotoxine aus Plasma entfernen, so ist zu berücksichtigen, daß diese aufgrund ihrer Phosphatgruppen im physiologischen Milieu (Blut-pH 7,36-7,44) geladen sind, so daß Adsorbermatrices mit kationischen Substituenten vorteilhafterweise eingesetzt werden. Besonders zweckmäßig sind Cellulose Beads, die mit kationischen Substituenten modifiziert sind, beispielsweise mit Diethylaminoethyl-, (DEAE)-Gruppen oder mit Polyethylenimin-Gruppen (PEI) modifiziert sind.

In einem ganz anderen klinischen Bereich, der Therapie von Hyperlipidämien, kann die erfindungsgemäße Technologie zur LDL-Adsorption eingesetzt werden. Auch hier können Cellulose-Beads, vorteilhafterweise mit einem Durchmesser um 5 µm zur Anwendung kommen.

Des weiteren lassen sich auch natürliche Zellen, wie Fibroplasten und dergleichen ohne weiteres für die erfindungsgemäßen Zwecke einsetzen, da sie keinerlei metabolische oder auch morphologische Schädigung nach einem 5-stündigen Durchlauf im Sekundärkreislauf aufweisen.

Festzustellen ist, daß es für den Erfolg der erfindungsgemäßen Anordnung nur darauf ankommt, daß die Reinigungssuspension bilanziert durch die Sekundärkammer des Membranfilters hindurchgepumpt wird, und zwar mit einem solchen Eingangsdruck, daß auf der einen Seite des Filters filtrierte Reinigungssuspension in die Blutkammer überführt wird und auf der anderen Seite das gleiche Volumen Plasma in die Filtratseite transportiert wird.

Die Zeichnung erläutert in Verbindung mit den nachstehend genannten Ausführungsbeispielen die Erfindung.

Es zeigen
- Abb. 1: eine Plasmaperfusionsanordnung,
- Abb. 2: eine erste Ausführungsform der erfindungsgemäßen Anordnung zur Elimination von Substanzen aus Blut,
- Abb. 3: eine zweite Ausführungsform der erfindungsgemäßen Anordnung in schematischer Darstellung,
- Abb. 4: die prinzipielle Anordnung eines Detektors zur Erfassung magnetisch aktiver Partikel,
- Abb. 5 und 6: im Querschnitt jeweils einen Hohlfadenmembranfilter mit gleicher Verteilung der Hohlfäden innerhalb des Gehäuses,
- Abb. 7: eine weitere Ausführungsform der erfindungsgemäßen Anordnung in schematischer Darstellung mit zwei Membranfilter und
- Abb. 8-10: jeweils Diagramme von Untersuchungen mit der erfindungsgemäßen Anordnung, wobei auf der Ordinate die Mengen der zu entfernenden Substanz und auf der Abszisse die Zeit aufgetragen ist.

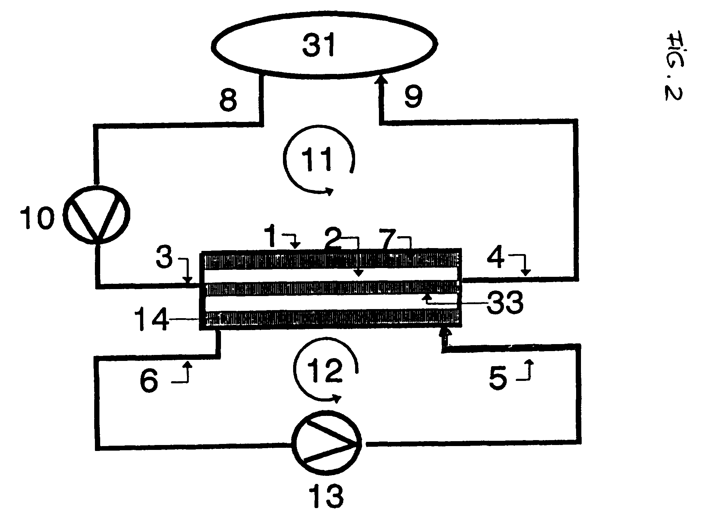

Abb. 2 zeigt die wesentlichen Elemente der Erfindung: Der Primärkreislauf 11 der Vorrichtung führt das venös oder arteriell entnommene Blut des Patienten 31 bzw. die zu reinigerde Flüssigkeit über einen Schlauch 8 zum Einlaß 3 der Primärseite 2 des Membranfilters 1 und weiter vom Auslaß 4 des Membranfilters 1 über einen Schlauch 9 zum Patienten bzw. Rezipienten zurück. Zur Aufrechterhaltung bzw. Erhöhung des Flusses im Primärkreislauf kann eine Pumpe 10 vorgesehen sein. Der Sekundärkreis 12 der Vorrichtung ist mit einer Reinigungssuspension gefüllt, die die Wirkstoffe bindenden Partikel 14 enthält, wobei die der Suspension zugrundeliegende Trägerflüssigkeit ihrerseits bindende Wirkung haben kann und darüber hinaus weitere Wirkstoffe, wie beispielsweise in an sich bekannter Weise Albumin, enthalten kann. Die Reinigungssuspension wird von einer Pumpe 13 durch den Sekundärkreislauf 12 getrieben, wobei diese Pumpe die Suspension bzw. ihre Partikel 14 weder mechanisch noch thermisch/chemisch schädigen darf, indem eine Pumpe mit in an sich bekannter Weise minimierter Scherbeanspruchung eingesetzt wird. Dies kann insbesondere mit Zentrifugalpumpen erreicht werden. Die Pumpe fördert die Reinigungsuspension durch den Einlaß 5 der Sekundärseite 7 in das Membranfilter 1, aus dem die Reinigungssuspension durch den Auslaß 6 wieder austritt. Aufgrund der Flüsse und Strömungswiderstände des Membranfilters kommt es sowohl auf der Primärseite 2 wie auch der Sekundärseite 7 zu Druckdifferenzen zwischen dem Einlaß 3 und Auslaß 4 der Primärseite wie auch dem Einlaß 5 und Auslaß 6 der Sekundärseite. Dadurch tritt eine lokal unterschiedliche transmembrane Druckdifferenz zwischen Primär- und Sekundärseite auf, die in Nähe von primärem Einlaß 3 und sekundärem Auslaß 6 von der Primärseite zur Sekundärseite gerichtet (als positiv bezeichnet) und in der Nähe von sekundären Einlaß 5 und primärem Auslaß 4 von der Sekundärseite zur Primärseite gerichtet (als negativ bezeichnet) ist. Damit kommt es zu einem Austausch von der die Filtermembran 33 durchdringenden Wirkstoffen enthaltende Flüssigkeit zwischen Primär- und Sekundärseite. Wird das zirkulierende Volumen der Reinigungssuspension des Sekundärkreislaufs 11 konstant gehalten, kann es im stationären Zustand zu keiner dauerhaften Volumensversehiebung kommen, da beispielsweise ein übermäßiger Übertritt von Flüssigkeit auf die Primärseite 2 zu einem Unterdruck auf der Sekundärseite 7 und damit wieder zu einem Übertritt von Flüssigkeit auf die Sekundärseite 7 führen würde. Das hat zur Folge, daß bei Konstanthaltung des Volumens im Sekundärkreislauf 12 der mittlere transmembrane Druck Null betragen muß.

Gleichzeitig lassen sich die lokalen transmembranen Druckdifferenzen sowohl durch eine Erhöhung des Flusses im Primärkreislauf 11 wie auch durch Erhöhung des Flusses im Sekundärkreislauf 12 erhöhen, was den Flüssigkeitsaustausch und damit die Eliminationsrate steigert. Die für eine wirksame Filtration vorgesehenen lokalen Druckgradienten liegen für medizinische Anwendungen zwischen 1 und 20 kPa. Bei Anwendung in der chemischen und biotechnologischen Trenntechnik können auch höhere lokale Druckgradienten vorgesehen sein. Anstatt des in der Abbildung 2 dargestellten Gegenstromprinzips, in dem die Flüsse auf Primärseite und Sekundärseite entgegengesetzt gerichtet sind, kann auch das Mitstromprinzip mit Flüssen gleicher Richtung zur Anwendung kommen.

Durch die Erzeugung dieser lokal unterschiedlichen Druckgradienten unterscheidet sich die neue Erfindung grundsätzlich von bekannten Geräten (z.B. BioLogicDT®) bei denen eine Suspension ohne ausreichende Geschwindigkeit zur Erzeugung derartiger Gradienten befördert wird.

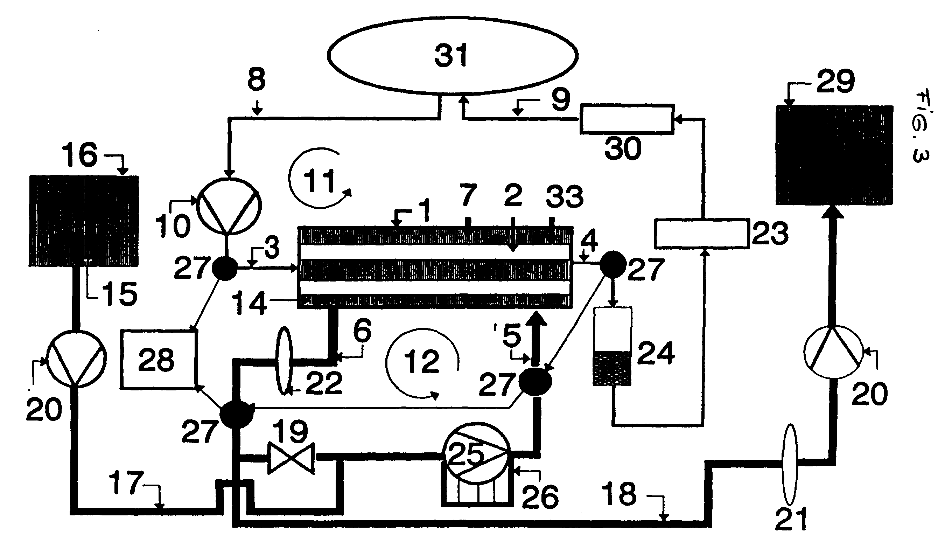

Abb. 3 zeigt die Vorrichtung mit möglichen Erweiterungen zur Verbesserung der Effizienz und Sicherheit.

Für den Ersatz von mit Wirkstoff gesättigter Reinigungssuspension kann eine Vorrichtung vorgesehen sein, die die Reinigungssuspension entweder kontinuierlich mit gleichmäßig laufenden Pumpsystemen oder diskontinuierlich erneuert. Um das Volumen im Sekundärkreislauf 12 konstant zu halten, kann dabei eine Doppelschlauchpumpe 20 oder ein anderes Pumpsystem mit zwei gleichartigen Förderungseinrichtungen vorgesehen sein, die neue Reinigungssuspension aus einem Reservoir 16 und einer Zuleitung 17 in den Sekundärkreislauf 12 pumpt und aus diesem die vorher verwendete Reinigungssuspension durch die Ableitung 18 in ein Reservoir 29 für verbrauchte Reinigungssuspension befördert. Anstelle der Doppelschlauchpumpe kann auch eine Anordnung von Ventilen im Zufluß 17 und Abfluß 18 vorgesehen sein, wobei zusätzlich eine einzelne Pumpe zur Beschleunigung des Austauschs vorgesehen sein kann. Zur Verminderung der Durchmischung von alter und neuer Reinigungssuspension kann darüber hinaus ein Sperrventil 19 vorgesehen sein, das während des dann diskontinuierlich durchzuführenden Austauschs eine Rezirkulation im Sekundärkreislauf 12 verhindert. Darüber hinaus können zur Verbesserung der Genauigkeit der Bilanzierung an sich bekannt Einrichtungen, wie Gewichts-Volumensmeßgeräte oder Bilanzkammern oder Zu- und Abflußpumpen mit gleichem Fördervolumen vorgesehen sein.

Um bei klinischer Anwendung eine Kontamination der Vorrichtung durch Wiederverwendung und dadurch folgende Schäden am Patienten 31 zu vermeiden, sind alle mit dem Blut oder der Reinigungssuspension in Kontakt kommenden Teile üblicherweise als Einmalartikel zur einmaligen Verwendung ausgelegt. Dies wird bei der Pumpe durch eine Aufteilung in einen einmal zu verwendenden Pumpenkopf 25 und einen wiederverwendbaren Antrieb 26 realisiert, die in bekannter Weise magnetisch gekoppelt oder über eine gedichtet Welle miteinander verbunden sind.

Um die Sicherheit des Patienten zu gewährleisten, ist bei einem Fehler des Membranfilters 1, wie beispielsweise Ruptur einer Membran, ein Eintritt von Partikeln 14 in den Blutkreislauf und damit den Primärkreislauf 11 zu vermeiden oder muß zumindest erkannt werden. Sind die Partikel 14 beträchtlich größer als die Blutbestandteile, kann dies in bekannter Weise durch ein Maschenfilter 24 realisiert werden, das die Partikel 14 an einem Eintritt in den Patienten 31 hindert. Sind die Partikel 14 von ähnlicher Größenordnung wie die Blutbestandteile, sollte ein Detektor 23 im Primärkreislauf vorgesehen sein, um die Vorrichtung abschalten zu können.

Dieser Detektor kann entweder in an sich bekannter Weise für eine Erkennung von Inhomogenitäten im Blut mit Ultraschall ausgelegt sein oder photooptisch sensitiv sein, wobei dann die Reinigungssuspension mit einem durch den Detektor erkennbaren Stoff (Farbstoff bzw. im sichtbaren und/oder unsichtbaren Bereich absorbierenden, luminiszierenden, fluoreszierenden oder phosphoriszierenden Stoff) gefärbt sein muß. Eine weitere Möglichkeit zum Ausfiltern von Partikeln 14 besteht im Anlegen eines Magnetfeldes bei Verwendung magnetisch aktivierbarer Anteile in den Partikeln 14. Das Magnetfeld treibt dann allfällig in den Primärkreislauf 11 gelangende Partikel 14 in die Fangvorrichtung 30, wo sie zurückgehalten werden. Eine derartige Fangvorrichtung kann auch im Sekundärkreislauf vorgesehen sein.

Weiters kann im Sekundärkreislauf 12 ein an sich bekannter Detektor 22 zur Erkennung von Erythrozyten und/oder von freiem Hämoglobin vorgesehen sein. Das Auftreten von Erythrozyten im Sekundärkreislauf würde auf Membranrupturen hinweisen, während freies Hämoglobin durch Zerstörung von Erythrozyten, beispielsweise aufgrund zu hoher Druckdifferenzen, entsteht. Um zu hohe Druckdifferenzen zu vermeiden, können in den Zuleitungen und/oder Ableitungen 3,4,5,6 des Membranfilters 1 Drucksensoren 27 vorgesehen sein. Diese können mit Anzeigegeräten verbunden sein, um dem Anwender Hinweise zur Einstellung der Pumpgeschwindigkeiten und über den Zustand des Membranfilters 1 (beispielsweise der Filtrationsrate) oder anderer Teile der Vorrichtung zu geben. Diese Einstellung der Pumpengeschwindigkeit kann auch mit einer automatischen Regeleinrichtung 28 zur Unterstützung des Anwenders versehen sein.

Um eine Ungleichverteilung der Partikel in der Reinigungssuspension und insbesondere im Reservoir 16 zu vermeiden, kann weiters eine Vibrations- oder Schwenkvorrichtung vorgesehen sein.

Für besondere Anwendungen, wie den Ersatz von eliminierten Substanzen durch therapeutisch indizierte Substanzen, wie beispielsweise Albumin, kann die Reinigungssuspension mit derartigen therapeutischen Substanzen beaufschlagt werden. Weiters kann durch ein absichtlich herbeigeführtes Ungleichgewicht zwischen Zufuhr und Abfuhr der Reinigungssuspension in den und vom Sekundärkreislauf 12 ein Flüssigkeitstransfer aus oder in den Patienten 31 herbeigeführt werden, wenn dies beispielsweise zur Volumenssubstitution von Blutverlusten oder zur Reduktion von Ödemen zweckmäßig erscheint.

In Abb. 4 ist eine Ausführungsform eines Detektors 23 dargestellt, der in Form einer Spule 36 um den Schlauch 9 herumgewickelt ist, um magnetisch aktive Partikel zu erfassen. Diese Partikel induzieren eine Spannung in der Spule 36, wenn sie diese passieren.

Zur Erhöhung der Empfindlichkeit kann der Schlauch bzw. die Leitung durch den Detektor 23 mit kleinerem Durchmesser als der Rest der Leitungen der Primärseite 11 ausgeführt sein, um durch die dann höhere Fließgeschwindigkeit eine Verbesserung der Empfindlichkeit zu erreichen.

Des weiteren kann die Empfindlichkeit verbessert und die Störsicherheit erhöht werden, wem mehrere derartige Spulen 36 hintereinander um den Schlauch herum bzw. neben dem Schlauch 9 zur Erfassung von Störfeldern vorgesehen sind.

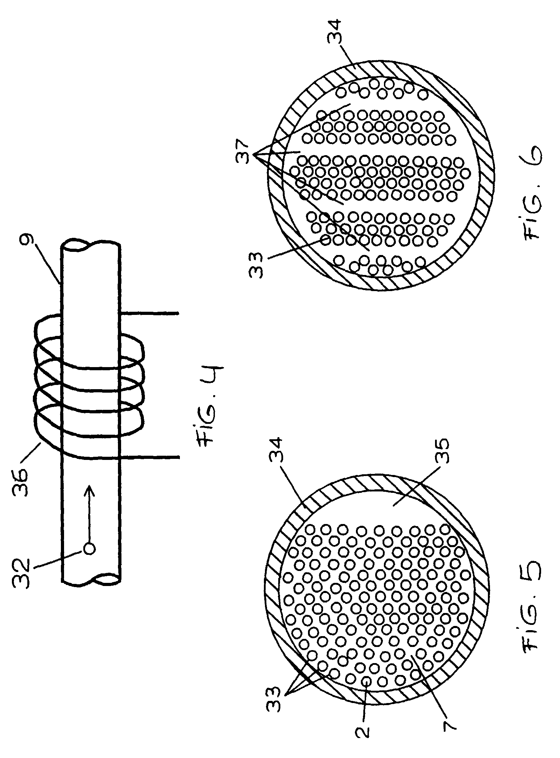

In Abb. 5 und 6 sind Ausführungsformen von Membranfiltern 1 im Querschnitt dargestellt, bei denen die hohlfaserartig ausgebildeten Membranen ungleichmäßig über die Innenfläche des Gehäuses 34 verteilt sind.

Gemäß Abb. 5 ist neben den Hohlfadenmembranen 33 eine einzelner freier über die gesamte Länge des Membranfilters 1 sich erstreckender Kanal 35 vorgesehen, der den freien Durchlauf der Reinigungssuspension gewährleistet.

Andererseits kann aber auch gemäß der in Abb. 6 gezeigten Ausführungsform eine Mehrzahl von freien Kanälen 37 zwischen den Hohlfadenmembranen 33 vorgesehen sein.

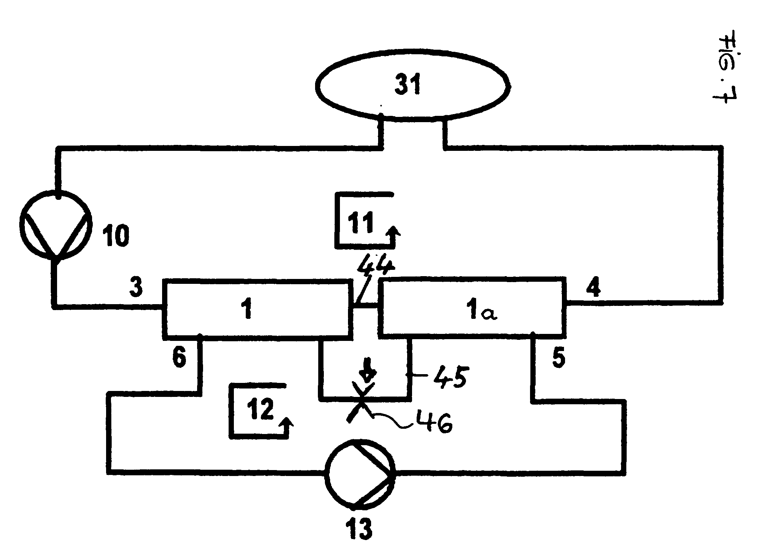

Eine besonders bevorzugte Ausführungsform der erfindungsgemäßen Anordnung ist in Abb. 7 dargestellt, die sich von der Ausführungsform gemäß Abb. 1 lediglich dadurch unterscheidet, daß anstelle von einem Membranfilter zwei Membranfilter 1,1a vorgesehen sind, die untereinander durch eine Verbindungsleitung 44 für den Primärkreislauf und eine Verbindungsleitung 45 für den Sekundärkreislauf in Strömungsverbindung sind. Besonders vorteilhaft ist in der letztgenannten Verbindungsleitung 45 eine regulierbare Klemme 46, was durch den Pfeil angezeigt ist, vorgesehen, so daß stromauf der Klemme ein bestimmter Überdruck einstellbar ist, der mit einem entsprechenden Unterdruck in dem Plasmafilter 1 wegen der Bilanzierung des Sekundärkreislaufs korreliert ist. Es kommt daher im Membranfilter 1 in vorbestimmter Weise zu einem Transfer eines bestimmten Plasmavolumens in die Sekundärkammer, wo dann die Elimination der zu entfernenden Substanzen stattfindet. In dem zweiten Membranfilter 1a wird dann das gleiche Volumen in den Blutkreislauf 4 zurückgeführt. Aufgrund der Regelbarkeit der Klemme 46 können daher exakt die zu behandelnden Volumina eingestellt werden.

Mit zwei hintereinandergeschalteten Filtern 1,1a gemäß der Ausführungsform von Abb. 7 kann die Eliminationsleistung gegenüber der Ein-Filter-Anordnung gemäß Abb. 2 noch verbessert werden.

### Behandlungsbeispiele 1-3

### Beispiel 1

In einem Batchversuch werden 18 ml porzines Plasma mit 2 ml Adsorbens in einen Kapillarplasmafilter auf Polypropylen-Basis (der Type P2 bzw. P2S der Firma Fresenius) mit einer Membranoberfläche von 0,5 m² vorgelegt. Der Sekundärkreislauf enthält Endotoxine in Form des Lipopolysaccharids von Pseudomonos Aeroginosa Serotype 10 Habs in einer Menge von 20 mg/ml Plasma. Der Gesamtproteingehalt beträgt 4,3 g/dl Plasma. Es wird bei 37°C inkubiert.

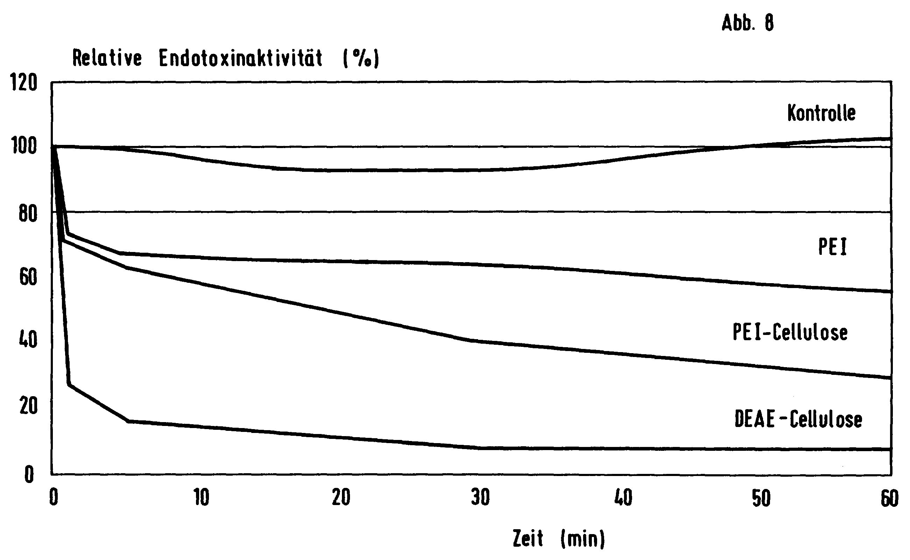

In Abb. 8 ist die relative Endotoxinaktivität in % gegenüber der Zeit aufgetragen, wobei die obere Linie als Kontrolle dient, d.h. der Sekundärkreislauf weist keine Adsorberteilchen auf. Die darunterliegenden Kurven beziehen sich auf Adsorberteilchen PEI (Polyethylenimin) bzw. PEI-Cellulose (Polyethylenimin-derivatisierte Cellulose-Beads) bzw. DEAE-Cellulose-Beads (Diethylaminoethyl-derivatisierte Cellulose Beads). Die Beads (ca. 10-20 µm) selber weisen eine hohe Adsorptionskapazität auf, was auf die hohe gutsmengen-bezogene Oberfläche der Kügelchen zurückzuführen ist.

Aus der Abb. 8 ist ersichtlich, daß in Abhängigkeit von den eingesetzten Beads mindestens die Hälfte der Endotoxinmenge eliminiert werden kann, wobei DEAE-Cellulose mehr als 90 % der Endotoxine innerhalb 10 Minuten entfernt.

### Beispiel 2

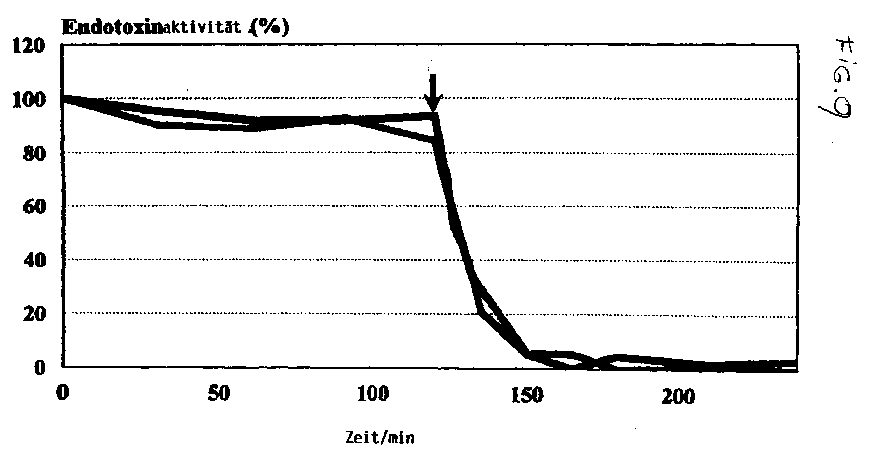

In Abb. 9 ist eine ähnliche Endotoxinelimination dargestellt, wobei zwei Filter entsprechend der Ausführungsform von Abb. 7 eingesetzt werden. Die gesamte Filteroberfläche beträgt 0,3 m², wobei Hohlfasermembranen aus Polypropylen eingesetzt werden.

Es werden auf der Primärseite 1,7 l Humanplasma eingesetzt, dem 40 mg/ml Endotoxin von Pseudomonos eroginosa bei 37°C inkubiert zugesetzt sind. Die volle Geschwindigkeit auf der Primärseite beträgt 0,2 l/min und auf der Sekundärseite 14 ml/min. Dem Sekundärkreislauf sind dabei 90 ml modifizierte PEI-Cellulose auf 120 ml isotonische Elektrolytlösung zugegeben.

Aus dem Diagramm ist ersichtlich, daß bei Zugabe der Microbeads (ca. 5-10 µm Durchmesser) die Endotoxinkonzentration steil abfällt, wobei dieser Abfall in zwei Tests dargestellt ist. Dabei ist ersichtlich, daß praktisch das gesamt Endotoxin innerhalb einer halben Stunde entfernt wird.

### Beispiel 3

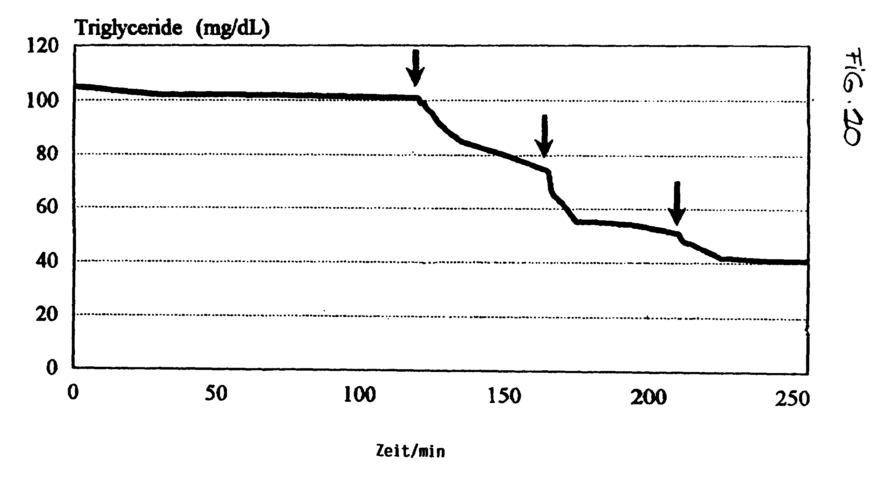

Es lassen sich mit dem erfindungsgemäßen Behandlungsverfahren nicht nur Endotoxine, sondern eine Vielzahl von anderen Partikeln durch die Wahl geeigneter Adsorbentien entfernen. In Abb. 10 ist nun die Entfernung von Triglyceriden (LDL) in der Zwei-Filter-Version gemäß Abb. 7 dargestellt.

Es kommen wieder Polypropylenhohlfasermembranen mit einer Gesamtoberfläche von 0,3 m² zum Einsatz. Des gleichen werden im Primärkreislauf 1,7 l Humanplasma mit einem Gesamtproteingehalt von 5 g/dl bei 37°C mit einer Förderrate von 200 ml/min gepumpt. Die Sekundärseite enthält als Adsorbens unmodifizierte Cellulose-Beads (R 94/29), isotonische Elektrolytlösung, wobei das Absorbens in Portionen von 140 ml bei 120, 160 bzw. 210 Minuten (vergl. die in der Abb. enthaltenen Pfeile) zugegeben wurde. Die Fördergeschwindigkeit auf der Sekundärseite beträgt dabei 14 l/min. Der Sekundärkreislauf enthält 300 ml Flüssigkeit.

Das LDL wird nach den üblichen Methoden bestimmt.

Aus Abb. 10 ist ersichtlich, wie der Gehalt an Triglyceriden im Primärkreislauf abnimmt in Abhängigkeit von der Zugabe der Absorberbeads.

## Patentansprüche

1. Anordnung zur Elimination von Substanzen aus Flüssigkeiten, insbesondere Blut, die mindestens einen an einen ersten Kreislauf (11), der die zu behandelnde Flüssigkeit aufweist, anschließbare Membranfilter (1, 1a) umfaßt, in dessen Sekundär- bzw. Filtratseite eine Absorbentien enthaltende Suspension vorliegt, um die durch die Membran (33) gelangenden Substanzen zu binden, wobei zur Erzielung einer Zirkulation der die Adsorbentien enthaltenden Suspension eine Pumpe (13, 25) im Sekundärkreislauf (12) vorgesehen und das Volumen des Sekundärkreislaufs (12) bilanziert gehalten ist, dadurch gekennzeichnet,
daß die Pumpe (13, 25) im Sekundärkreislauf als ein Mittel zur Erzeugung regional auftretender unterschiedlicher positiver und negativer Transmembrandrücke am Membranfilter (1, 1a) ausgebildet ist, wobei die Pumpe (13, 25) mit einer Förderrate betreibbar ist, daß es zu einem Austausch von der den Membranfilter (1, 1a) durchdringende Substanzen enthaltenden Flüssigkeit zwischen Primär- und Sekundärkreislauf (11, 12) kommt.

2. Anordnung nach Anspruch 1, dadurch gekennzeichnet, daß zwei hintereinandergeschaltete Membranfilter (1, 1a) vorgesehen sind.

3. Anordnung nach Anspruch 1, dadurch gekennzeichnet, daß die zweite Pumpe (13, 25) im Gegenstrom arbeitet.

4. Anordnung nach Anspruch 1 oder 2, dadurch gekennzeichnet, daß die zweite Pumpe (13, 25) eine Förderleistung von 0,5-6 l/min, vorzugsweise 1-3 l/min aufweist.

5. Anordnung nach einem der Ansprüche 1-3, dadurch gekennzeichnet, daß die zur Förderung der Suspension vorgesehene Pumpe (13, 25) eine Zentrifugalpumpe ist, um durch die dadurch bewirkte niedrige mechanische Scherbelastung der suspendierten adsorbierenden Partikel die mechanische Schädigung derselben zu minimieren.

6. Vorrichtung nach einem der Ansprüche 1 bis 4, dadurch gekennzeichnet, daß zum Austausch der Reinigungssuspension eine Pumpenanordnung (20) mit zwei gleichartigen Fördereinrichtungen vorgesehen ist, die gleiche Mengen an Reinigungssuspension zu- und abführen.

7. Vorrichtung nach den Ansprüchen 1 bis 5, gekennzeichnet durch eine Ventilanordnung zur Konstanthaltung des Volumens, wobei zusätzlich vorzugsweise eine Pumpe zur Beschleunigung des Austauschs und/oder ein Sperrventil (19) zur Verhinderung von Durchmischung von verbrauchter und neuer Reinigungssuspension vorgesehen sind.

8. Vorrichtung nach den Ansprüchen 1 bis 6, dadurch gekennzeichnet, daß sämtliche mit der Reinigungssuspension in Kontakt kommende Teile zur einmaligen Verwendung bestimmt sind, wobei die verwendeten Pumpen aus einem wiederverwendbaren Antrieb (26) und einem zur einmaligen Verwendung bestimmten Pumpenkopf (25) bestehen.

9. Vorrichtung nach den Ansprüchen 1 bis 7, dadurch gekennzeichnet, daß im Primärkreislauf (11) nach dem Auslaß (4) der Primärseite (2) des Membranfilters (1) ein Detektor (23) zur Erkennung von Defekten des Filters, vorzugsweise Ultraschalldetektor, zwischengeschaltet ist, der Partikel, die in die Flüssigkeit des Primärkreislaufes (1) gelangt sind, erkennt.

10. Vorrichtung nach den Ansprüchen 1 bis 9, dadurch gekennzeichnet, daß ein Maschenfilter (24) nach dem Auslaß (4) der Primärseite (2) des Membranfilters (1) geschaltet ist.

11. Vorrichtung nach Anspruch 10, dadurch gekennzeichnet, daß im Primärkreislauf (11) nach dem Auslaß (4) der Primärseite (2) des Membranfilters (17) ein magnetisches Feld aufbringbar ist und die Partikel (14) der Reinigungssuspension eine magnetisch aktive Substanz enthalten, so daß bei einem Defekt des Membranfilters (1) Partikel, die auf die Primärseite übertreten, durch eine Fangvorrichtung (30) abgefangen werden.

12. Vorrichtung nach den Ansprüchen 1 bis 11, dadurch gekennzeichnet, daß bei den Ein- und/oder Auslässen (3, 4, 5, 6) des Membranfilters (1) Drucksensoren (27) angebracht sind.

13. Vorrichtung nach den Ansprüchen 1 bis 12, dadurch gekennzeichnet, daß die Reinigungssuspension zusätzlich zu den pathophysiologisch relevanten Substanzen bindenden Partikeln (14) Wirkstoffe enthält, die durch den Austauschvorgang dem Hauptsystem zuführbar sind.

14. Vorrichtung nach den Ansprüchen 1 bis 13, gekennzeichnet durch eine Steuereinrichtung, die eine unterschiedlich große Zufuhr und Abfuhr von Reinigungssuspension in und vom Sekundärkreis (12) ermöglicht.

15. Vorrichtung nach Anspruch 2, dadurch gekennzeichnet, daß in die Verbindungsleitung (45) zwischen den Filtratkammern der Membranfilter (1) und (1a) eine regulierbare Klemme (46) eingeschaltet ist.

## Claims

1. Arrangement for the elimination of substances from liquids, in particular from blood, said arrangement comprising at least one membrane filter (1, 1a) connectable to a first circuit (11) which contains the liquid to be treated, in the secondary side or, respectively, filtrate side of said membrane filter being comprised a suspension that contains adsorbents in order to bind the substances passing the membrane (33), wherein a pump (13, 25) is provided in the secundary circuit (12) to effect circulation of said suspension containing the adsorbents and wherein the volume of the secundary circuit (12) is kept bilanced, said arrangement being **characterized in that** the pump (13, 25) in the secondary circuit is developped so as to constitute a means for the generation of regionally occurring different positive and negative transmembrane pressures at the membrane filter (1, 1a), whereat the pump (13, 25) can be operated with such a delivery capacity that, between the primary and secondary circuit (11, 12), liquid is exchanged containing the substances that pass the membrane filter (1, 1a).

2. Arrangement according to claim 1, characterized in that two membrane filters (1, 1a) are provided that are connected in series.

3. Arrangement according to claim 1, characterized in that the second pump (13, 25) operates in reverse flow sense.

4. Arrangement according to claim 1 or 2, characterized in that the second pump (13, 25) has a delivery capacity of 0.5-6 l/min, preferably of 1-3 l/min.

5. Arrangement according to one of claims 1 to 3, characterized in that the pump (13, 25) provided for the delivery of the suspension is a centrifugal pump, to the end that, by means of the low mechanical shearing forth effected therewith, the mechanical damage of the suspended absorbing paticles is minimized.

6. Device according to one of claims 1 to 4, characterized in that, for an exchange of the cleaning suspension, a pump arrangement (20) is provided having two delivery equipments of the same kind which supply and remove equal amounts of the cleaning suspension.

7. Device according to claims 1 to 5, characterized by a valve arrangement to keep the volume constant, wherein preferably a pump for the enhancement of the exchange and/or a non-return valve (19) to prevent the mingling of used and new cleaning suspension are additionally provided.

8. Device according to claims 1 to 6, characterized in that all pieces contacting the cleaning suspension are destined for single-use, whereas the employed pumps are composed of a reusable drive (26) and a pump head (25) for single-use.

9. Device according to claims 1 to 7, characterized in that, after the outlet (4) on the primary side (2) of the membrane filter (1), a detector (23) is interconnected in the primary circuit for the detection of damages of the filter, said detector detecting particles penetrated into the liquid of the primary circuit (1).

10. Device according to claims 1 to 9, characterized in that a meshed filter (24) is interconnected after the outlet (4) on the primary side (2) of the membrane filter (1).

11. Device according to claim 10, characterized in that, after the outlet (4) on the primary side (2) of the membrane filter (17), a magnetic field can be applied to the primary circuit (11) and that the parrticles (14) of the cleaning suspension contain a magnetically active substance so that, in case of a damage of the membrane filter (1), the particles passed over into the primary side are intercepted by an intercepting device (30).

12. Device according to claims 1 to 11, characterized in that manometric sensors (27) are mounted at the inlets and /or outlets (3, 4, 5, 6) of the membrane filter (1).

13. Device according to claims 1 to 12, characterized in that, in addition to the particles (14) which bind pathophysiologically relevant substances, the cleaning suspension comprises agents that can be supplied to the main system by means of the exchange process.

14. Device according to claims 1 to 13, characterized by a control equipment that enables the cleaning suspension to be supplied and removed into and out of the secondary circuit (12).

15. Device according to claim 2, characterized in that an adjustable clamp (46) can be interconnected in the connection line (45) between the filtrate chambers of the membrane filters (1) and (1a).

## Revendications

1. Disposition pour éliminer des substances de liquides, notamment du sang, comportant au moins un filtre à diaphragme (1, 1a) que l'on peut joindre à un premier cycle (11) contenant le liquide à traiter, où du côté secondaire ou, respectivement, du côté de filtrat dudit filtre à diaphragme se trouve une suspension contenant des adsorbants pour la liaison des substances parvenant à travers le diaphragme (33), où l'on a prévu une pompe (13, 25) dans le cycle secondaire (12) pour obtenir une circulation de la suspension contenant les adsorbants et où le volume du cycle secondaire (12) est maintenu bilancé,
ladite disposition étant caractérisée en ce que la pompe (13, 25) dans le cycle secondaire est développée en tant que moyens pour la génération de pressions transmembranes différentielles positives et négatives se présentant régionalement au filtre à diaphragme (1, 1a), et que l'on peut conduire la pompe (13, 25) d'une telle capacité de refoulement que, entre le cycle primaire est secondaire (11, 12), un échange du liquide est exercé qui contient des substances traversant le filtre à diaphragme (1, 1a).

2. Disposition d'après la revendication 1, caractérisée en ce que l'on a prévu deux filtres à diaphragme (1, 1a) installés en série.

3. Disposition d'après la revendication 1, caractérisée en ce que la deuxième pompe (13, 25) fonctionne à refoulement inversé

4. Disposition d'après la revendication 1 ou 2, caractérisée en ce que la deuxième pompe (13, 25) a une capacité de refoulement de 0,5 à 6 l/min, de préférence de 1 à 3 l/min.

5. Disposition d'après une des revendications 1 à 3, caractérisée en ce que la pompe (13, 25) prévue pour le transport de la suspension est une pompe centrifuge afin que, par la sollicitation au cisaillement mécanique réduit des particules adsorbantes tenues en suspension en effectuée, la détérioriation mécanique desdites particules soit minimalisé.

6. Dispositif d'après une des revendications 1 à 4, caractérisé en ce que, pour l'échange de la suspension à nettoyer, on a prévu une disposition de pompage (20) ayant deux dispositions de refoulement de la même sorte qui alimentent et emmènent une quantité égale de suspension à nettoyer.

7. Dispositif d'après les revendications 1 à 5, caractérisé par une disposition de vanne pour garder le volume constant, où l'on a prévu de préférence une pompe supplémentaire pour l'accélération de l'échange et/ou une soupape supplémentaire (19) pour empêcher que la suspension à nettoyer employée est mélangée avec la suspension fraîche.

8. Dispositif d'après les revendications 1 à 6, caractérisé en ce que tous les pièces mises en contact avec la suspension à nettoyer sont destinées pour usage unique où les pompes employées sont composées d'une motorisation réutisable(26) et d'un piston de pompe (25) destiné pour usage unique.

9. Dispositif d'après les revendications 1 à 7, caractérisé en ce que, dans le cycle primaire (11), après la sortie (4) du côté primaire (2) du filtre à diaphragme (1), on a inserté un détecteur (23) pour l'identification de défauts, de préférence un détecteur à ultrasons, qui percevoit des particules parvenus dans le liquide du cycle primaire (1).

10. Dispositif d'après les revendications 1 à 9, caractérisé en ce qu' un filtre de maille (24) est inserté après la sortie (4) du côté primaire (2) du filtre à diaphragme (1).

11. Dispositif d'après la revendication 10, caractérisé en ce que, après la sortie (4) du côté primaire (2) du filtre à diaphragme (17), on peut appliquer un champ magnétique dans le cycle primaire (11) et que les particules (14) de la suspension à nettoyer comprennent une substance magnétiquement active pour que des particules transférés au côté primaire soient recueillis par un dispositif d'arrêt (30), si le filtre à diaphragme (1) est défaut.

12. Dispositif d'après les revendications 1 à 11, caractérisé en ce que l'on a monté des détecteurs manométriques (27) aux entrées et/ou aux sorties (3, 4, 5, 6) du filtre à diaphragme (1).

13. Dispositif d'après les revendications 1 à 12, caractérisé en ce que, en plus des particules (14) recueillant les substances pathophysiologiquement importantes, la suspension à nettoyer comprend des agents que l'on peut alimenter au systéme principal par le processus d'échange.

14. Dispositifd'après les revendications 1 à 13, caractérisé par un appareil de commande permettant une alimentation et un enlèvement dans une mesure différente de la suspension à nettoyer dans et du cycle primaire (12).

15. Dispositif d'après la revendication 2, caractérisé en ce que, entre les chambres de filtrat des filtres à diaphragme (1) et (1a), on a inserté une pince réglable (46) dans la conduite de connexion (45).
